# NEUE EUROPÄISCHE PATENTSCHRIFT

(11) **EP 0 312 913 B2**
(45) Veröffentlichungstag und Bekanntmachung des Hinweises auf die Entscheidung über den Einspruch: **20.12.1995**
(45) Hinweis auf die Patenterteilung: 29.01.1992
(21) Anmeldenummer: 88117022.9
(22) Anmeldetag: 13.10.1988
(51) Int. Cl.: A61K 31/425, A61K 31/55, A61K 31/34

(54) **Verwendung von paf-Acether-Antagonisten zur Herstellung eines Arzneimittels und Verfahren zu deren Wirksamkeitsbestimmung**
Use of paf-antagonists for the preparation of a medicament and method for the determination of their binding-activity
Usage des paf-antagonistes pour la préparation d'un medicament et méthode pour la détermination de leur activité bloquante

(30) Priorität: 20.10.1987 DE 3735525
(43) Veröffentlichungstag der Anmeldung: 26.04.1989
(73) Patentinhaber: Korth, Ruth-Maria, Dr. med, D-80639 München (DE)
(72) Erfinder: Korth, Ruth-Maria, Dr. med, D-80639 München (DE)
(74) Vertreter: Brose, D. Karl, Dipl.-Ing.

(56) Entgegenhaltungen:
- EP-A- 176 927
- EP-A- 194 416
- EP-A- 254 245
- EP-B- 230 942
- DE-A- 3 435 973
- DE-A- 3 502 392
- DE-A- 3 635 771
- DE-A- 3 635 771
- DE-A- 3 636 306
- DE-A- 3 710 921
- DE-A- 3 735 525
- GB-A- 2 162 062
- BIOCHEMICAL AND BIOPHYSICAL RESEARCH COMMUNICATIONS Band 128, Nr. 2, 30 April 1985., Seiten 972-979; SAN-BAO H. et al.: "Specific binding sites for platelet activating factor in human lung tissues"
- Braguet et al., Pharmacological Research Communications, vol. 18, suppl. 1986
- Handley et al., Drug Development Research 7, 361 (1986)
- "PAF-Acether Antagonists in Experimental Arterial Thrombosis" Braquest et al., New Horizons Platelet Activating Factor Research (1985)
- "PAF-Acether in chronic arthritis 1987. E.R.Pettipher et al. Agents and Actions Vol. 21, 98 (1987)
- Oedeme. Bjork et al., Acta Physiol. Scand. 119, 305-308, 1983. "Bakterielle Erkrankungen", septischer Schock.
- Sanchez-Crespo et al., Pharmacol. Res. Comm. 18, Suppl. 1986.
- Doebber et al., Biochem. Biophys. Res. Comm. 127, pp. 799-808, 1985.
- "Cerebrale Erkrankungen". Stroke Plotkine et al., Prostaglandin-Meeting, June 1986, Abstr. Book 1986, p.300
- Kochanek et al., 2 Int. Conf. on Paf, Gatlinburg, 1986, Abstr. book, p. 118
- Spinnewyn et al., Prostaglandins 34, p. 337-349, 1987. "Metastasierung", Tumore.
- Record et al., Cancer 71, p. 223, 1984.
- Grignani et al., Haematoligica 71, p. 245-255, 1986
- Tanaka et al., Invasion Metastasis 6, p. 209-224, 1986. Gefässverengung.
- Siren et al., 2nd. Int. Conf. Paf, Gatlinburg, 1986, Abstr. Book, p. 88
- Casals-Stenzel et al., Br. J.Pharmacol. 1987
- Camussi et al. Journal de Pharmacologie 14 (Suppl.1) 1983
- Bourgain et al. Journal de Pharmacologie 14 (Suppl.) 1983
- R. Korth et al. Comparison of Human Endothelial Cells in Cultur and Platelets for Tritium. PAF-Acether Binding 71st. Annual Meeting of the Federation of American Societies for Experimental Biology (1987).

## Beschreibung

Die Erfindung bezieht sich auf die Verwendung von paf-Acether-Antagonisten zur Herstellung eines Arzneimittels und ein Verfahren zur Bestimmung von deren Wirksamkeit.

Paf-Acether (platelet-activating-factor) ist seiner chemischen Struktur nach 1-0-Alkyl-2-acetyl-sn-glyceryl-3--phosphorylcholin (J. Exp. Med., 136: 1356-1377, 1972; J. Biochem., 254: 9355-9358, 1979). Es ist bekannt, daß paf-Acether starke inflammatorische und hypotensive Eigenschaften besitzt und die koronare Vasokonstriktion sowie die akute Ödembildung in der Haut induziert. Es sind paf-Acether-Rezeptoren an Blutplättchen, polymorphonuklearen Neutrophilen und Membranpräparationen menschlichen Lungengewebes beschrieben worden (J. Immunol., 129: 1637-1641, 1984; Thromb. Res. 41: 699-706, 1986; Immunology 48: 141, 1983; Biochem. Biophys. Res. Commun. 128: 972, 1985). Da Ginkgolide die Blutplättchenaggregation inhibieren, die durch paf-Acether hervorgerufen wird, wird in Eur. J. Pharmacol. 152: 101-110; 1988, vorgeschlagen, Ginkgolide in Arzneimitteln zur Behandlung von Krankheiten als Folge der Blutplättchenaggregration einzusetzen.

Das Endothel bildet eine zellige Auskleidung, die das Gewebe schützt. Beim Durchbrechen der Endothelzellen-Barriere treten eine Reihe von Erkrankungen auf. So kann Flüssigkeit aus dem Gewebe austreten, was zur Ödembildung führen kann. Auch können bakterielle und zerebrale Erkrankungen, Entzündungen und Allergien Folge der Herabsetzung der Endothelzellen-Barriere sein. Durch Angriff der Herzkranzgefäße können bei Abbau der Endothelzellen-Barriere ferner spastische Koronarverengungen hervorgerufen werden. Da das Endothel vor einer Absiedlung von erkrankten Gewebeteilen schützt, wirkt es einer Metastasierung entgegen. Auch führen Liäsonen des Endothels zu einem gesteigerten Stoffeinstrom, der Reaktionen hervorruft, die zur Arteriosklerose führen können. Wie nach DE-A-3710921 festgestellt wurde, weisen die Endothelzellen Bindungsstellen oder Rezeptoren für paf-Acether auf.

Überraschenderweise wurde nun festgestellt, daß Substanzen, die die paf-Acether-Bindungsstellen an Endothelzellen inhibieren (paf-Acether-Antagonisten) sich zur Vorbeugung von Erkrankungen eignen, die durch den Abbau der Endothelzellen-Barriere hervorgerufen werden, und zwar von Ödemen, dem wichtigsten Symptom von Entzündungen, und Allergien, einschließlich Asthma, bakteriellen und zerebralen Erkrankungen sowie Gefäßverengungen, insbesondere am Magen-Darmtrakt, des Gehirns und des Herzens, z. B. spastischen Koronarverengungen oder Arteriosklerose. Sie sind ferner zur Herabsetzung einer Metastasierung geeignet.

Die paf-Acether-Bindungsstellen inhibierende Substanz kann dabei ein hydrophiles Triazolothieno-diazepin sein. Daneben haben sich Ginkgolide sowie paf-Acether-Analoge wie CV 3988 als geeignet erwiesen. Triazolothieno-diazepine sind in Br. J. Pharmac. (1987) 90: S. 139-146 beschrieben, Ginkgolide in "Blood and Vessel" (1985), Nr. 16: S. 558-572). Von den hydrophilen Triazolothieno-diazepin-Verbindungen sind insbesondere WEB 2086 und 2098 geeignet. Von den Ginkgoliden zeigen BN 52020, BN 52021 sowie ein Gemisch aus BN 52020, BN 52021 und BN 52022, welches als BN 52063 bezeichnet wird, die besten Resultate.

Das Gingkolid-Gemisch BN 52063 zeichnet sich neben seiner hohen Wirksamkeit durch seine relativ leichte Herstellbarkeit aus, da es bei der Aufarbeitung der Gingkolide als Gemisch anfällt, also nicht in einzelne Komponenten zerlegt werden muß.

Erfindungsgemäß werden inbesondere WEB 2086 und WEB 2098, CV 3988 und BN 52020, BN 52021 und BN 52063 verwendet, um eine Durchbrechung der Endothelzellenbarriere durch paf-Acether sowie durch paf-Acether stimulierte Zellen zu verhindern.

CV 3988 hat die chemische Bezeichnung rac-3(N-n-Octadecyl-carbamoyloxy)-2-methoxypropyl-2-thiazolylethylphosphat, WEB 2086 die Bezeichnung 3-(4-(2-Chlorophenyl)-9-methyl-6H-thieno(3,2,-f) (1,2,4) triazolo-(4,3-a)- (1,4) diazepin -2yl)-1-(4-morpholinyl)-1-propanon, WEB 2098 die Bezeichnung 3-(4-(2-Chlorophenyl)-9-cyclopropyl-6H-thieno (3,2-f)-(1,2,4)triazolo-(4,3-a) (1,4)diazepin-2yl)-1-(4-morpholinyl) -1-propanon, BN 52020 die Bezeichnung 9H-1, 7a-(Epoxymethano)-1H, 6aH-cyclopenta*[*c*]*furo*[*2,3-b*]* furo-*[*3',2':3,4*]* cyclopenta *[*1,2-d*]*furan-5,9,12(4H)-trion, 3-tert-butylhexahydro-4, 7b-dihydroxy-8-methyl, BN 52021 die Bezeichnung 9H-1, 7a-(Epoxymethano)-1H,6aH-cyclopenta *[*c*]* furo *[*2,3-b*]* furo *[*3',2' :3 ,4*]* cyclopenta*[1*,2-d*]*furan-5,9,12(4H)-trion, 3-tert-butylhexahydro-4, 4b-11-trihydroxy-8-methyl und BN 52022 die Bezeichnung 9H-1,7a-(Epoxymethano)-1H, 6aH-cyclopenta*[*c*]*furo *[3'*,2':3,4*]* cyclopenta*[*1,2-d*]* furan-5,9,12(4H)-trion, 3-tert-butylhexahydro-2,4,7b,11-tetrahydroxy-8-methyl.

Die paf-Acether-Bindungsstellen inhibierenden Substanzen können z.B. durch Injektion, aber auch oral, perkutan und durch Inhalation verabreicht werden.

Um die Wirkung der Substanzen auf ihre paf-Acether-Rezeptorantagonistische Aktivität schnell und einfach zu testen, also z.B. im Screening-Verfahren wirksame paf-Acether-Rezeptor-Antagonisten in Bezug auf Endothelzellen aufzufinden, die dann zur Behandlung der erwähnten Erkrankungen in Betracht gezogen werden können, wird erfindungsgemäß vorzugsweise wie folgt vorgegangen:
a) man kultiviert Endothelzellen,
b) die Endothelzellen werden gewaschen,
c) man versetzt eine vorgegebene Menge der Endothelzellen mit einer vorgegebenen Menge paf-Acether und des zu bestimmenden markierten Antagonisten,
d) man versetzt eine vorgegebene Menge der Endothelzellen mit einer vorgegebenen Menge markiertem Antagonisten,
e) die Endothelzellen werden von dem Gemisch c) und d) jeweils abgetrennt,
f) man mißt jeweils die Menge des an die Endothelzellen gebundenen markierten Antagonisten und
g) aus dem Verhältnis der Menge des markierten Antagonisten der gemäß c) in Gegenwart von paf-Acether an die Endothelzellen gebunden ist und der Menge des markierten Azitagonisten, der gemäß d) ohne paf-Acether an die Endothelzellen gebunden ist, bezogen auf die gleiche Menge Endothelzellen, wird die Wirksamkeit des paf-Acether-Antagonisten bestimmt.

Die Kultivierung der Endothelzellen gemäß Schritt a) erfolgt erfindungsgemäß vorzugsweise in einem serumhaltigen, insbesondere Kalbsserum-haltigen Kulturmedium. Es ist jedoch auch ein serumfreies Kulturmedium verwendbar.

Dabei werden in dem Kulturmedium vorzugsweise konfluente Endothelzellen gezüchtet, welche im allgemeinen am Boden des Kulturgefäßes anwachsen.

Um Serum und damit paf-Acether abbauende Enzyme, wie Acetylhydrolase, zu entfernen, werden die Endothelzellen dann gemäß Schritt b) des erfindungsgemäßen Verfahrens gewaschen. Dabei wird eine gepufferte, isotonische Waschflüssigkeit verwendet, die vorzugsweise delipidiertes Serumalbumin, wie Humanserumalbumin (HSA) oder Rinderserumalbumin (BSA) enthält, einschließlich endotoxinfreiem Serumalbumin.

Alsdann wird gemäß den Schritten c) und d) erfindungsgemäß eine Bindungsstudie an den Endothelzellen mit markiertem paf-Acether und dem zu bestimmenden Antagonisten einerseits und markiertem paf-Acether, jedoch ohne Antagonisten, andererseits, durchgeführt. Als markierter paf-Acether wird dabei vorzugsweise radioaktiv markierter paf-Acether, beispielsweise tritiummarkierter paf-Acether, verwendet.

Die Bindungsstudien werden vorzugsweise bei einer Temperatur von weniger als 20°C durchgeführt, vorzugsweise bei 2-6°C, damit die in den Endothelzellen enthaltenen Enzyme, wie Phospholipasen oder Acetylhydrolase, zu keinem Abbau des paf-Acethers führen. Die Inkubationszeit beträgt vorzugsweise 10 bis 30 Std..

Da paf-Acether als Phospholipid nicht in Wasser geht, werden die Bindungsstudien gemäß den Schritten c) und d) ferner vorzugsweise in Gegenwart eines delipidierten Serumalbumins, wie HSA oder BSA, einschließlich endotoxinfreiem Serumalbumin, durchgeführt, wobei vorzugsweise Calcium- und Magnesiumionen zugesetzt werden.

Das Serumalbumin hat dabei die Aufgabe, den an die Endothelzellen unspezifisch, also nicht an die Rezeptoren gebundenen paf-Acether und Antagonisten zu binden, d.h. von den Endothelzellen zu entfernen.

Zwischen den Schritten e) und f) werden die konfluenten Endothelzellen von ihrer Unterlage (Kulturschale) und voneinander gelöst.

Dazu werden vorzugsweise noch weitere Schritte durchgeführt, um Endothelzellen zu erhalten, die im wesentlichen maximal spezifisch an die Rezeptoren gebundenen paf-Acether bzw. Antagonisten aufweisen. Zu diesem Zweck werden die Endothelzellen zunächst vorzugsweise mit einer isotonischen Flüssigkeit, also insbesondere einer physiologischen Kochsalzlösung versetzt, wodurch Calcium- und Magnesiumionen sowie unspezifisch gebundener paf-Acether und Antagonist von den Endothelzellen abgetrennt werden.

Dann wird vorzugsweise in einem zweiten Waschschritt eine auf 5°C oder darunter, vorzugsweise auf etwa 0°C gekühlte isotonische Flüssigkeit den Endothelzellen zugesetzt, welche vorzugsweise EDTA enthält. Durch die niedrige Temperatur und das die Calciumionen in den Endothelzellen bindende EDTA ziehen sich die konfluenten Endothelzellen zusammen, wodurch sie sich von dem Kulturgefäß oder der sonstigen Unterlage, aber auch voneinander lösen, d.h. die konfluente Endothelzellenschicht wird zerstört. Zugleich gehen durch die niedrige Temperatur der Waschflüssigkeit unspezifisch gebundener paf-Acether und Antagonist von den Endothelzellen leichter ab.

Um die so behandelten Endothelzellen von dem flüssigen Medium abzutrennen, werden sie bevorzugt filtriert. Sie sind nämlich ausgezeichnet filtrierbar, da bei dem geschilderten Lösen die Endothelzellen nicht beschädigt und schon gar nicht zerstört werden. Zugleich stellt das Filtrieren ein relativ scharfes und damit sehr wirksames Verfahren zur Abtrennung des restlichen unspezifisch gebundenen markierten paf-Acethers von den Endothelzellen dar.

Anschließend wird die Menge des an die Endothelzellen (spezifisch) gebundenen markierten paf-Acethers bestimmt, wobei bei Verwendung von radioaktiv markiertem paf-Acether lediglich die Radioaktivität der filtergebundenen Endothelzellen gemessen wird. Die filtergebundene Radioaktivität in Abwesenheit von Endothelzellen wird von diesen Werten abgezogen.

Durch Eichkurven, die mit unterschiedlichen Mengen des Antagonisten gemäß dem Schritt c) erhalten werden, kann so die Wirksamkeit des Antagonisten als 50%iger Hemmwert erhalten werden, d.h. als die Menge des Antagonisten, die bezogen auf eine vorgegebene Menge Endothelzellen, zu einer 50%igen Hemmung der reversiblen paf-Acether-Bindung führt.

Das erfindungsgemäße Verfahren wurde mit Erfolg mit folgenden paf-Acether-Rezeptor-Antagonisten getestet: hydrophiles Triazolothieno-diazepin WEB 2086 und 2098, dem Gingkolid BN 52020, BN 52021 sowie einem Gemisch aus BN 52020, BN 52021 und BN 52022 sowie mit CV 3988.

Als paf-Acether-Rezeptor-Antagonisten kommen erfindungsgemäß auch gegen diese Rezeptoren gerichtete monoklonale Antikörper in Betracht. Die Antikörper können auch in markierter oder fluoreszierender Form Verwendung finden. In diesem Fall wird vor der Bindungsstudie gemäß den Schritten c) und d) eine Vorinkubation der Endothelzellen mit überschüssigem Antikörper durchgeführt. Die Bindungsstudien gemäß c) und d) können auch mit radioaktiv markierten oder fluoreszierenden paf-Acether-Antagonisten oder Analoge durchgeführt werden, die dann durch nicht markierten paf-Acether aus der Bindung verdrängt werden. Desgleichen kann beim erfindungsgemäßen Verfahren der paf-Acether durch paf-Acether-Analoge ersetzt sein und der paf-Acether-Antagonist durch Analoge solcher Antagonisten.

Das erfindungsgemäße Verfahren dient nicht nur zur Bestimmung von Antagonisten der Rezeptoren für paf-Acether als solchen, sondern auch für die Bestimmung von paf-Acether-Komplexen, insbesondere dem paf-Acether/Lipoprotein-Komplex, der als "PEAK X" bezeichnet wird, welcher in Fed. Proc. 46, 1987 (3), Seite 1468, beschrieben wird.

Das nachstehende Beispiel dient der weiteren Erläuterung der Erfindung.

### 1. Herstellung der Endothelzellen.

Menschliche Endothelzellen wurden von der Nabelschnurvene isoliert (J. Clin Invest., 52: 2745-2756, 1973). D.h. die Vene wurde mit einer Kanüle versehen und mit 0,1 %igen Collagenaselösungen gefüllt (Typ I, Sigma, St. Louis, MO, USA). Nach einer 10 Minuten langen Inkubation bei 37°C wurden die gelösten Zellen herausgespült und durch Zentrifugieren gesammelt. Die Zellen wurden in einem Hams-F-12-Medium mit 10 % hitzeinaktiviertem foetalen Kalbsserum (FCS Hyclone, Logan, Utah, USA), 1 % Ultroser SF (IBF, Villeneuve la Garenne, Frankreich) und 90 »g/ml Heparin ergänzt mit 50 U/ml Penicillin und 50 »g/ml Streptomycin, resuspendiert (Science 222: 623-625, 1983). Die Zellen wurden in einer 25 cm² Kulturschale inkubiert (Primaria, Falcon, Labware, Oxnard, Kalifornien, USA), und zwar eine Stunde. Die nichthaftenden Zellen wurden dann vorsichtig weggewaschen und die haftenden Zellen wurden weiter in frischem Kulturmedium kultiviert, wobei ein Mediumwechsel alle 2 Tage vorgenommen wurde. Nachdem die Kulturen Konfluenz (3 bis 5 Tage) erreicht hatten, wurden die Zellen geerntet, indem sie kurz Trypsin-EDTA (Gibco, Paisley, Schottland) ausgesetzt wurden und in eine 35 mm Kulturschale (Primaria, Falcon) mit einem 1 : 3 bis 1 : 5 Splitverhältnis gegeben wurden. Die Zellen wurden dann weiter in einem Hams-F-12-Medium mit 15 % FCS, 1 % Ultroser SF und 90 »g/ml Heparin wachsen gelassen, wobei ein Mediumwechsel alle zwei bis drei Tage vorgenommen wurde. Die Zellen erreichten eine Konfluenz nach 4 bis 6 Tagen, wobei die Zahl der Zellen 5,2 ± 0, 15 x 10⁵ je Schale betrug. Die Zellen des ersten Durchgangs wurden während der gesamten Untersuchung verwendet. Die Kulturen wurden als Endothelzellen aufgrund morphologischer Kriterien und durch indirekte Immunfluoreszenz unter Verwendung eines spezifischen Antiserums für humanes Faktor VIII-Antigen (Nordic Immunol., Tilburg, Niederlande), welches ein übliches Markierungsmittel für endotheliale Zellen ist, bestimmt. Die Endothelzellkulturen enthielten keine Monocyten/Makrophagenhaltigen Zellen aufgrund morphologischer Kriterien und durch indirekte Immunfluoreszenz unter Verwendung monoklonaler Antikörper für Alpha₂-Makroglobulin.

### 2. Paf-Acether-Bindung an Endothelzellen.

Von den konfluenten, also zusammengewachsenen menschlichen Endothelzellen wurde zweimal sorgfältig das Kulturmedium mit Tyrodes Puffer abgewaschen, welcher 0,25 % Rinderserumalbumin (BSA) enthielt. Zu den konfluenten endothelialen Zellen wurden 900 »l Tyrodes Puffer (pH 7,4), 0,25 % BSA (Sigma), 1,3 mM Ca²⊕, 100 »l ³H-paf-Acether (0,065 nM) (1-0-³H-Octadecyl-2-acetyl-sn-glyceryl-3-phosphorylcholin 80 Ci/mmol, Amersham, Bucks, Großbritannien) mit und ohne 500 nM unmarkiertem paf-Acether (1-0-Octadecyl-2-acetyl-sn-glyceryl-3-phosphorylcholin) (Bachem, Bubendorf, Schweiz) in unterschiedlichen Zeitabständen (3, 5, 15, 30 Min.) gegeben.

Bei einem zweiten Experiment wurden unterschiedliche Konzentrationen von ³H-paf-Acether (0,0325 - 0,65 nM) in Abwesenheit oder Gegenwart von unmarkiertem paf-Acether oder dessen Homologe (3-0-Hexadecyl-2-acetylglyceryl-1-phosphorylcholin) (500 nM) 30 Minuten inkubiert, wie vorstehend beschrieben.

In weiteren Versuchen wurden die paf-Acether-Antagonisten BN 52021 (60, 10, 6 »M) (IHB-IPSEN, Le Plesses-Robinson, Frankreich), CV 3988 (30, 10, 3 »M) (Takeda Chemical Ind., Osaka, Japan) oder Vehikel (Dimethylsulfoxid oder isotonische NaCl-Lösung) zusammen mit 0,65 nM ³H-paf-Acether zu konfluenten Endothelzellen gegeben.

Nach 30 Minuten wurde die freie Aktivität zweimal von den konfluenten Endothelzellen mit Tryodes Puffer (pH 6,4), welcher BSA enthielt, gewaschen, und dann einmal mit kalter isotonischer NaCl-Lösung. Die Zellen wurden durch Zugabe von kalter isotonischer EDTA-NaCl-Lösung (5 mM) getrennt, wonach bei 0 - 4°C 30 - 60 Minuten inkubiert wurde. Die gelösten Zellen wurden von dem Medium durch Vakuumfiltration mit einem Millipore-Vakuumsystem (Molsheim, BRD) abgetrennt. Der Inkubationspuffer wurde ebenfalls filtriert, um einen Verlust an Zellen während des Waschvorgangs nach dem Bindungsversuch zu vermeiden. Die Filter wurden zweimal mit 10 ml kaltem Tyrodes Puffer gewaschen und die Radioaktivität, die an die Filter gebunden war, wurde unter Standardbedingungen in PCS (Amersham) bestimmt. Die filtergebundene Radioaktivität in Abwesenheit von Endothelzellen wurde abgezogen von der Radioaktivität in Gegenwart von Endothelzellen. Die gebundene Radioaktivität wurde in fmol ³H-paf-Acether, gebunden an 5,20 ± 0, 15 x 10⁵ Endothelzellen, berechnet.

Nach einer Inkubation von 30 Minuten mit steigenden Konzentrationen von paf-Acether-Antagonisten wurden die Zellen durch kalte EDTA-NaCl-Lösung freigesetzt und dann durch Vakuumfiltration abgetrennt. Die ³H-paf-Acether-Bindung wurde in fmol, gebunden an 5,2 ± 0,15 x 10⁵ Endothelzellen berechnet.

In der nachstehenden Tabelle sind die Ergebnisse der spezifischen ³H-paf-Acether-Bindung in fmol, an 5,2 ± 0,15 x 10⁵ konfluente Endothelzellen wiedergegeben.

**Tabelle 1**

| | CV 3988 | | BN 52021 |
|---|---|---|---|
| | fmol | | fmol |
| 3 »M | 2,26 ± 1,4 | 6 »M | 1,6 ± 1,4 |
| 10 »M | 2,70 ± 1,9 | 10 »M | 4,8 ± 3,5 |
| 30 »M | 4,40 ± 1,4 | 60 »M | 3,9 ± 3,1 |

D.h. die paf-Acether-Bindung an 5,2 x 10⁵ Endothelzellen vermindert sich bei einem Zusatz von 3 »M CV 3988 um 2,26 ± 1,4 fmol, bei einem Zusatz von 6 »M BN 52021 um 1,6 ± 1,4 fmol usw.. D.h. CV 3988 stellt einen wirksameren Antagonisten als BN 52021 dar.

Statt einer Inkubationszeit von 30 Min. bei 20°C kann auch 20 Std. bei 4°C inkubiert werden, wobei die Antagonisten vorzugsweise 15 Min. vorinkubiert werden. Dadurch wird u.a. eine Metabolisierung der Liganden verhindert.

Die paf-Acether-Bindung an Endothelzellen nach dem vorstehenden Versuch unter Ziffer 2 wurde wiederholt, außer daß statt BN 52021 bzw. CV 3988 das hydrophile Triazolothieno-diazepin WEB 2086 (1, 10, 100, 1000 nM) verwendet wurde.

Die Ergebnisse sind in der nachstehenden Tabelle 2 wiedergegeben.

**Tabelle 2**

| WEB 2086 | |
|---|---|
| 1 nM | 0,66 ± 0,2 fmol |
| 10 nM | 1,5 ± 1,1 fmol |
| 100 nM | 2 ± 1,1 fmol |
| 1000 nM | 3 ± 0,7 fmol |

D.h. die paf-Acether-Bindung an 5,2 x 10⁵ Endothelzellen vermindert sich bei einem Zusatz von 1 nM WEB 2086 um 0,66 ± 0,2 und bei 1000 nM um 3 ± 0,7 fmol.

## Patentansprüche

1. Verwendung von paf-Acether-Antagonisten zur Herstellung eines Arzneimittels für die Vorbeugung von Arteriosklerose, Ödemen, bakteriellen und zerebralen Erkrankungen, der Metastasierung sowie von Gefäßverengungen durch Verhinderung der Herabsetzung der Endothelzellenbarriere.

2. Verwendung nach Anspruch 1, dadurch gekennzeichnet, daß der paf-Acether-Antagonist ein hydrophiles Triazolothieno-diazepin, ein Ginkgolid, CV 3988 oder ein monoklonaler Antikörper ist, der gegen endotheliale paf-Acether-Rezeptoren gerichtet ist.

3. Verwendung nach Anspruch 2, dadurch gekennzeichnet, daß das hydrophile Triazolothieno-diazepin WEB 2086 oder WEB 2098 und das Ginkgolid BN 52020, BN 52021 oder ein Gemisch aus BN 52020, BN 52021 und BN 52022 ist.

4. Verfahren zur Bestimmung der Wirksamkeit von paf-Acether-Antagonisten nach den Ansprüchen 1 bis 3, gekennzeichnet durch folgende Schritte:
a) man kultiviert Endothelzellen,
b) die Endothelzellen werden gewaschen,
c) man versetzt eine vorgegebene Menge der Endothelzellen mit einer vorgegebenen Menge paf-Acether und des zu bestimmenden radioaktiv markierten oder fluoreszierenden Antagonisten,
d) man versetzt eine vorgegebene Menge der Endothelzellen mit einer vorgegebenen Menge radioaktiv markierten oder fluoreszierenden Antagonisten,
e) die Endothelzellen werden von dem Gemisch c) und d) jeweils abgetrennt,
f) man mißt jeweils die Menge des an die Endothelzellen gebundenen radioaktiv markierten oder fluoreszierenden Antagonisten und
g) aus dem Verhältnis der Menge des radioaktiv markierten oder fluoreszierenden Antagonisten, der gemäß c) in Gegenwart von paf-Acether an die Endothelzellen gebunden ist und der Menge des radioaktiv markierten oder fluoreszierenden Antagonisten, der gemäß d) ohne paf-Acether an die Endothelzellen gebunden ist, bezogen auf die gleiche Menge Endothelzellen, wird die Wirksamkeit des paf-Acether-Antagonisten bestimmt.

5. Verfahren nach Anspruch 4, dadurch gekennzeichnet, daß beim Schritt a) konfluente Endothelzellen kultiviert werden, welche nach der Abtrennung der Endothelzellen gemäß e) gelöst werden.

6. Verfahren nach Anspruch 5, dadurch gekennzeichnet, daß das Lösen der Endothelzellen in einer auf 5°C oder darunter gekühlten isotonischen Flüssigkeit erfolgt.

7. Verfahren nach Anspruch 6, dadurch gekennzeichnet, daß die isotonische Flüssigkeit EDTA enthält.

8. Verfahren nach einem der Ansprüche 4 bis 7, dadurch gekennzeichnet, daß dem Endothelzellen, paf-Acether und radioaktiv markierten oder fluoreszierenden Antagonisten enthaltendem Medium gemäß c) und dem Endothelzellen und radioaktiv markierten oder fluoreszierenden Antagonisten enthaltenden Medium gemäß d) jeweils Serumalbumin zugesetzt wird.

9. Verfahren nach einem der Ansprüche 4 bis 8, dadurch gekennzeichnet, daß das Waschen der Endothelzellen gemäß b) mit einer isotonischen Flüssigkeit erfolgt, die Serumalbumin enthält.

10. Verfahren nach einem der Ansprüche 4 bis 9, dadurch gekennzeichnet, daß die Abtrennung gemäß e) durch Filtrieren erfolgt.

11. Verfahren nach Anspruch 4, dadurch gekennzeichnet, daß die Endothelzellen vor den Schritten c) und d) mit monoklonalen Antikörpern, die gegen endotheliale paf-Acether-Rezeptoren gerichtet sind, vorinkubiert werden.

## Claims

1. The use of paf-acether-antagonists for the production of a medicine for prevention of arteriosclerosis, oedemas, bacterial and cerebral diseases, metastatic diseases as well as vascular contraction by prevention of weakening of the endothelium cell barrier.

2. The use according to claim 1, wherein the paf-acether antagonist is a hydrophilic triazolo-thieno-diazepine, a Ginkgolide, CV 3988 or a monoclonal antibody directed against endothelial paf-acether-receptors.

3. The use according to claim 2, wherein the hydrophilic triazolo-thieno-diazepine is WEB 2086 or WEB 2098 and the Ginkgolide is BN 52020, BN 52021 or a mixture of BN 52020, BN 52021 and BN 52022.

4. A procedure for determination of efficacy of paf-acether-antagonists according to claims 1 to 3, characterized by the following steps:
a) Endothelium cells are cultivated,
b) endothelium cells are washed,
c) a given quantity of endothelium cells is mixed with a given quantity of paf-acether and of the radioactively labelled or fluorescent antagonist to be determined,
d) a given quantity of endothelium cells is mixed with a given quantity of the radioactively labelled or fluorescent antagonist,
e) the endothelium cells are separated from the mixture c) and d) in each case,
f) the quantity of the radioactively labelled or fluorescent antagonist bound to the endothelium cells is measured in each case, and
g) the efficacy of the paf-acether antagonist is determined from the relationship between the quantity of the radioactively labelled or fluorescent antagonist which is bound to the endothelium cells according to c) in the presence of the paf-acether on the one hand, and the quantity of the radioactively labelled or fluorescent antagonist which is bound to the endothelium cells according to d) in the absence of paf-acether on the other hand, related to the same amount of endothelium cells.

5. The procedure according to claim 4, characterized in that confluent endothelium cells are cultivated in step a) which are detached after the separation of the endothelium cells according to step e).

6. The procedure according to claim 5, characterized in that the endothelium cells are detached in an isotonic liquid cooled to 5°C or lower.

7. The procedure according to claim 6, characterized in that the isotonic liquid contains EDTA.

8. The procedure to any one of the claims 4 to 7, characterized in that serum albumin is added to the medium containing endothelium cells, paf-acether and radioactively labelled or fluorescent antagonist in accordance with c) and to the medium containing endothelium cells and radioactively labelled or fluorescent antagonist in accordance with d) in each case.

9. The procedure to any one of the claims 4 to 8, characterized in that the washing of the endothelium cells in accordance with b) is done with an isotonic liquid, containing serum albumin.

10. The procedure according to any one of the claims 4 to 9, characterized in that the separation in accordance with e) is carried out by means of filtering.

11. The procedure according to claim 4, characterized in that before steps c) and d) the endothelium cells are preincubated in the presence of monoclonal antibodies which are directed against endothelial paf-acether receptors.

## Revendications

1. Usage des paf-acéther-antagonistes pour la préparation d'un médicament pour la prévention de l'artériosclérose, des oedèmes, des maladies bactérielles et cérébrales, des métastases, ainsi que des rétrécissements des vaisseaux comme conséquence de la diminution de la barrière de cellules endothéliales.

2. Usage selon la revendication 1, caractérisée en ce que le paf-acéther-antagoniste est une triazolothiénodiazépine hydrophile, un ginkgolide, CV 3988, ou un anticorps monoclonal, orienté pour agir contre les récepteurs paf-acéther endothéliaux.

3. Usage selon la revendication 2, caractérisée en ce que la triazolothiéno-diazépine hydrophile est le WEB 2086 ou le WEB 2098, et le ginkgolide est le BN 52020, BN 52021 ou un mélange des BN 52020, BN 52021 et du BN 52022.

4. Procédé de détermination de l'activité bloquante des paf-acéther-antagonistes selon les revendications 1 à 3, caractérisé par les étapes suivantes:
a) on cultive les cellules endothéliales,
b) on lave les cellules endothéliales,
c) on mélange une quantité prédéterminée de cellules endothéliales à une quantité prédéterminée de paf-acéther et de l'antagoniste marqué radioactivement ou fluorescent, à déterminer,
d) on mélange une quantité prédéterminée de cellules endothéliales à une quantité prédéterminée de l'antagoniste marqué radioactivement ou fluorescent,
e) on sépare les cellules endothéliales respectivement du mélange c) et du mélange d),
f) on mesure chaque fois la quantité de l'antagoniste marqué radioactivement ou fluorescent, qui a été combinée aux cellules endothéliales, et
g) à partir du rapport entre la quantité de l'antagoniste marqué radioactivement ou fluorescent, qui a été combinée aux cellules endothéliales selon c) en présence de paf-acéther et la quantité d'antagoniste marqué radioactivement ou fluorescent, qui a été combinée aux cellules endothéliales selon d) sans la présence de paf-acéther, en référence à la même quantité de cellules endothéliales, on détermine l'efficacité du paf-acéther-antagoniste.

5. Méthode selon la revendication 4, caracterisée en ce que, lors de l'étape a), on cultive des cellules endothéliales confluentes, dissoutes après la séparation des cellules endothéliales selon e).

6. Méthode selon la revendication 5, caractèrisèe en ce que la dissolution des cellules endothéliales s'effectue dans un liquide isotonique refroidi à la température de 5°C ou au-dessous.

7. Méthode selon la revendication 6, caractérisée en ce que le liquide isotonique contient de l'EDTA.

8. Méthode selon l'une des revendications 4 à 7, caractérisée en ce que l'on ajoute du sérum-albumine d'une part au milieu contenant les cellules endothéliales, le paf-acéther et des antagonistes marqués radioactivement ou fluorescents selon c) et d'autre part au milieu contenant les cellules endothéliales, l'antagoniste marqué radioactivement ou fluorescent selon d).

9. Méthode selon l'une des revendications 4 à 8, caractérisée en ce que le lavage des cellules endothéliales selon b) s'effectue à l'aide d'un liquide isotonique qui contient du sérum-albumine.

10. Méthode selon l'une des revendications 4 à 9, caractérisée en ce que la séparation selon e) s'effectue par filtration.

11. Méthode selon le revendication 4, caractérisée en ce que l'on préincube les cellules endothéliales avant les étapes c) et d), avec des anticorps monoclonaux, orientés contre les paf-acéther-récepteurs endothéliaux.
